# EUROPEAN PATENT APPLICATION

(11) **EP 3 305 203 A1**
(43) Date of publication of application: **11.04.2018**
(21) Application number: 16799809.5
(22) Date of filing: 11.05.2016
(51) Int. Cl.: A61B 8/12, H04R 17/00

(54) **ULTRASONIC PROBE**

(30) Priority: 25.05.2015 JP 2015105542
(71) Applicant: Olympus Corporation, Hachioji-shi Tokyo 192-8507 (JP)
(72) Inventor: SATO, Sunao, Tokyo 192-8507 (JP)
(74) Representative: Schicker, Silvia
(86) International application number: PCT/JP2016/063967
(87) International publication number: WO 2016/190101

(57) **Abstract**

An ultrasound probe according to the present invention includes an acoustic matching layer, a plurality of piezoelectric elements, and a structural member. The acoustic matching layer has a sheet-shaped member whose both ends are bonded together by an adhesive to define a cylindrical shape and is configured to adjust acoustic impedance of ultrasound. The plurality of piezoelectric elements is arranged along a circumferential direction on an inner periphery of the acoustic matching layer and configured to emit ultrasound in accordance with an input of an electrical signal and configured to convert ultrasound that has entered from outside, into an echo signal. The structural member has a hollow disc shape, provided on at least one end side in a longitudinal direction orthogonal to the circumferential direction of the acoustic matching layer, and at least a portion of an outer side surface is fixed inside the acoustic matching layer. The structural member has a water repellent portion on the outer side surface such that the water repellent portion faces, in a radial direction, a gap between the both ends of the acoustic matching layer.

## Description

### Field

The present invention relates to an ultrasound probe including an ultrasound transducer for emitting ultrasound to an observation target, receiving an ultrasound echo reflected from the observation target, converting the ultrasound echo into an echo signal, and outputting the echo signal.

### Background

Ultrasound is applied in some cases for observing characteristics of a body tissue or material as an observation target. Specifically, an ultrasound observation apparatus performs predetermined signal processing onto an ultrasound echo received from an ultrasound transducer configured to transmit and receive an ultrasound, whereby information related to the characteristics of the observation target can be obtained.

The ultrasound transducer includes a plurality of piezoelectric elements that converts an electrical pulse signal into an ultrasound pulse (acoustic pulse), emits the ultrasound pulse to the observation target, converts an ultrasound echo reflected from the observation target into an electrical echo signal, and outputs the echo signal. The ultrasound echo is obtained from the observation target, for example, by arranging the plurality of piezoelectric elements in a predetermined direction, electronically switching the elements related to transmission and reception, or delaying transmission and reception of each of the elements.

There are a plurality of types of known ultrasound transducers having different transmission and reception directions of ultrasound beams, such as a convex ultrasound transducer, a linear ultrasound transducer, and a radial ultrasound transducer. Among these, a radial ultrasound transducer includes a plurality of piezoelectric elements arranged in a circumferential direction and radially emits an ultrasonic beam (for example, see Patent Literature 1). Such a radial ultrasound transducer is manufactured by first deforming a sheet-shaped acoustic matching layer on which the plurality of piezoelectric elements is arranged, into a cylindrical shape along the arrangement direction of the piezoelectric elements, and thereafter bonding ends in a circumferential direction with each other using adhesive.

### Citation List

### Patent Literature

Patent Literature 1: JP 63-14623 B

### Summary

### Technical Problem

In a manufacturing process of the radial ultrasound transducer, however, there is a possibility that, when the ends of the acoustic matching layer are joined with each other, adhesive might flow into portions between the piezoelectric elements from joined portions and might deteriorate acoustic performance of the ultrasound transducer.

The present invention has been made in view of the foregoing, and an object of the present invention is to provide an ultrasound probe capable of suppressing deterioration in acoustic performance of the radial ultrasound transducer.

### Solution to Problem

In order to solve the above described problem and achieve the object, an ultrasound probe according to the invention includes: an acoustic matching layer having a sheet-shaped member whose both ends are bonded together by an adhesive to define a cylindrical shape and configured to adjust acoustic impedance of ultrasound; a plurality of piezoelectric elements arranged along a circumferential direction on an inner periphery of the acoustic matching layer and configured to emit ultrasound in response to an input of an electrical signal and configured to convert ultrasound that has entered from outside, into an echo signal; a structural member having a hollow disc shape and provided on at least one end side of the acoustic matching layer in a longitudinal direction orthogonal to the circumferential direction, at least a part of an outer side surface of the structural member being fixed inside the acoustic matching layer. The structural member has a water repellent portion on a part of the outer side surface such that the water repellent portion faces, in a radial direction, a gap between the both ends of the acoustic matching layer.

The ultrasound probe according to the invention further includes a second structural member having a hollow disc shape and provided on the other end side in the longitudinal direction of the acoustic matching layer, an outer side surface of the second structural member being fixed inside the acoustic matching layer. The second structural member has a water repellent portion on the outer side surface so as to face the gap in the radial direction.

In the ultrasound probe according to the invention, the water repellent portion is formed by one of a surface having water repellent coating or a surface having a surface roughness structure.

In the ultrasound probe according to the invention, the water repellent coating is one of fluorine coating and hydrophobic silica particle coating.

In the ultrasound probe according to the invention, the surface roughness structure is a double roughness structure.

In the ultrasound probe according to the invention, the water repellent portion is exposed to outside by the gap.

In the ultrasound probe according to the invention, the structural member is configured such that the outer side surface other than the water repellent portion is bonded to the acoustic matching layer via an adhesive.

### Advantageous Effects of Invention

According to the present invention, it is possible to suppress deterioration in acoustic performance of the radial ultrasound transducer.

### Brief Description of Drawings

FIG. 1 is a diagram schematically illustrating an endoscope system according to an embodiment of the present invention.
FIG. 2 is a perspective view schematically illustrating a configuration of a distal end of an insertion unit of an ultrasound endoscope according to the embodiment of the present invention.
FIG. 3 is a diagram illustrating a configuration of an ultrasound transducer according to the embodiment of the present invention.
FIG. 4 is a diagram illustrating a method for manufacturing the ultrasound transducer according to the embodiment of the present invention.
FIG. 5 is a diagram illustrating a method for manufacturing the ultrasound transducer according to the embodiment of the present invention.
FIG. 6 is a diagram illustrating a method for manufacturing the ultrasound transducer according to the embodiment of the present invention.
FIG. 7 is a diagram illustrating a method for manufacturing the ultrasound transducer according to the embodiment of the present invention.
FIG. 8 is a diagram illustrating a method for manufacturing the ultrasound transducer according to the embodiment of the present invention.
FIG. 9 is a diagram illustrating a method for manufacturing the ultrasound transducer according to the embodiment of the present invention.
FIG. 10 is a diagram illustrating a method for manufacturing the ultrasound transducer according to the embodiment of the present invention.
FIG. 11 is a diagram illustrating a method for manufacturing the ultrasound transducer according to the embodiment of the present invention.
FIG. 12 is a diagram illustrating a method for manufacturing the ultrasound transducer according to the embodiment of the present invention.
FIG. 13 is a diagram illustrating a method for manufacturing the ultrasound transducer according to the embodiment of the present invention.
FIG. 14 is a diagram illustrating a method for manufacturing the ultrasound transducer according to the embodiment of the present invention.
FIG. 15 is a diagram illustrating a method for manufacturing the ultrasound transducer according to the embodiment of the present invention.
FIG. 16 is a diagram illustrating a method for manufacturing the ultrasound transducer according to the embodiment of the present invention.

### Description of Embodiments

Hereinafter, modes for carrying out the present invention (hereinafter, referred to as embodiment(s)) will be described with reference to the drawings. The present invention is not limited by the embodiments. The same reference signs are used to designate the same elements throughout the drawings.

### (Embodiments)

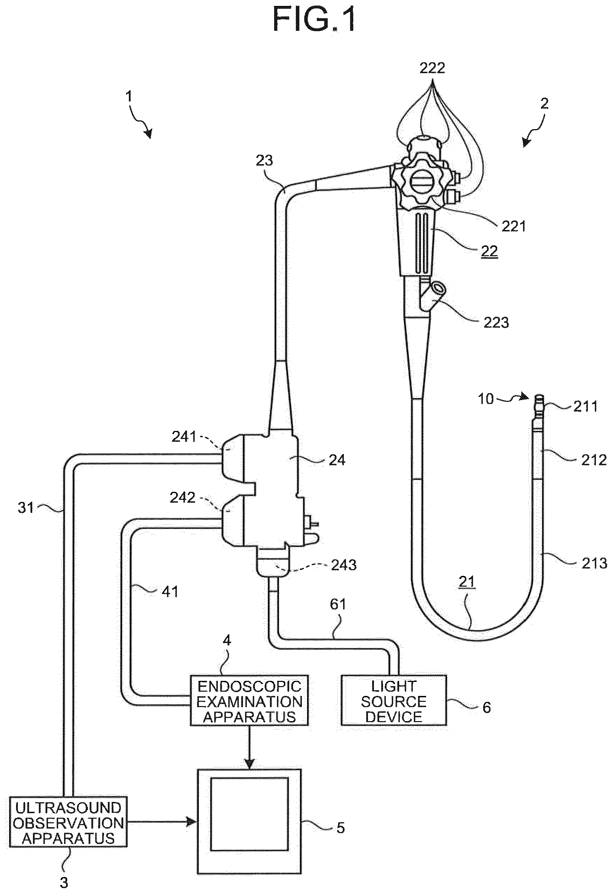

FIG. 1 is a diagram schematically illustrating an endoscope system according to an embodiment of the present invention. An endoscope system 1 is a system for performing ultrasound diagnosis of internal portions of a subject such as a human using an ultrasound endoscope. As illustrated in FIG. 1, the endoscope system 1 includes an ultrasound endoscope 2, an ultrasound observation apparatus 3, an endoscopic examination apparatus 4, a display device 5, and a light source device 6.

The ultrasound endoscope 2 includes, on its distal end portion, an ultrasound transducer. The ultrasound transducer converts an electrical pulse signal received from the ultrasound observation apparatus 3 into an ultrasound pulse (acoustic pulse) and emits it to the subject. The ultrasound transducer also converts an ultrasound echo reflected from the subject into an electrical echo signal expressed by a voltage change and outputs the signal. The configuration of the ultrasound transducer will be described below.

The ultrasound endoscope 2 typically includes imaging optics and imaging sensors. The ultrasound endoscope 2 can be inserted into gastrointestinal tracts (esophagus, stomach, duodenum, and large intestine) or respiratory organs (trachea, bronchus) of the subject and can capture the gastrointestinal tract and the respiratory organs. Moreover, it is possible to capture their surrounding organs (pancreas, gall bladder, bile duct, biliary tract, lymph nodes, mediastinal organs, blood vessels, or the like) using ultrasound. The ultrasound endoscope 2 includes a light guide that guides illumination light emitted to the subject at the time of optical imaging. The light guide is configured such that a distal end portion thereof reaches a distal end of an insertion unit of the ultrasound endoscope 2 into the subject, while a proximal end portion thereof is connected to the light source device 6 that generates illumination light.

As illustrated in FIG. 1, the ultrasound endoscope 2 includes an insertion unit 21, an operating unit 22, a universal cable 23, and a connector 24. The insertion unit 21 is a portion to be inserted into the subject. As illustrated in FIG. 1, the insertion unit 21 includes a rigid member 211, a bending portion 212, and a flexible tube portion 213. The rigid member 211 includes an ultrasound transducer 10 provided on a distal end side. The bending portion 212 is bendable and coupled to the proximal end side of the rigid member 211. The flexible tube portion 213 is coupled to the proximal end side of the bending portion 212. While specific illustration is omitted herein, the insertion unit 21 internally includes a light guide, a plurality of signal cables, and an insertion passage for treatment tools. The light guide transmits illumination light supplied from the light source device 6. The plurality of signal cables transmits various signals. The insertion passage for treatment tools is used for inserting treatment tools.

The ultrasound transducer 10 is a radial transducer. The ultrasound transducer 10 performs electronic scanning by arranging the plurality of piezoelectric elements in the circumferential direction, electronically switching piezoelectric elements related to transmission and reception, or delaying transmission and reception of each of the piezoelectric elements. The configuration of the ultrasound transducer 10 will be described below.

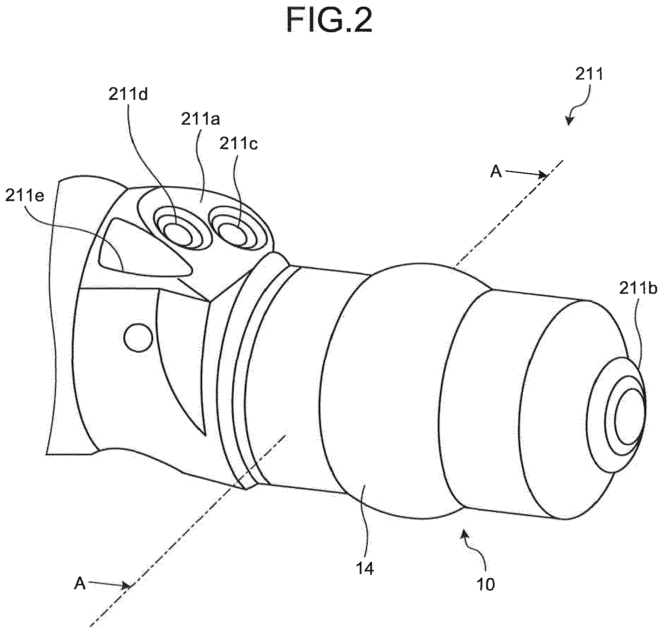

FIG. 2 is a perspective view schematically illustrating a configuration of a distal end of an insertion unit of the ultrasound endoscope according to the embodiment, being a diagram illustrating a configuration of the rigid member 211. The rigid member 211 includes the ultrasound transducer 10 and a proximal end portion 211a. The ultrasound transducer 10 is provided at the distal end side. The proximal end portion 211a is provided on the proximal end side of the ultrasound transducer 10 and includes an inclined surface. Moreover, a balloon locking unit 211b is formed at the distal end of the rigid member 211. The balloon locking unit 211b is capable of locking a balloon. The proximal end portion 211a includes an illumination lens 211c, an objective lens 211d, and a treatment tool protruding port 211e. The illumination lens 211c collects illumination light and emits the illumination light to the outside. The objective lens 211d is a portion of the imaging optics, and configured to take in the light from the outside. The treatment tool protruding port 211e communicates with the insertion passage for treatment tools formed inside the insertion unit 21, and configured to cause the treatment tool to protrude from the distal end of the insertion unit 21.

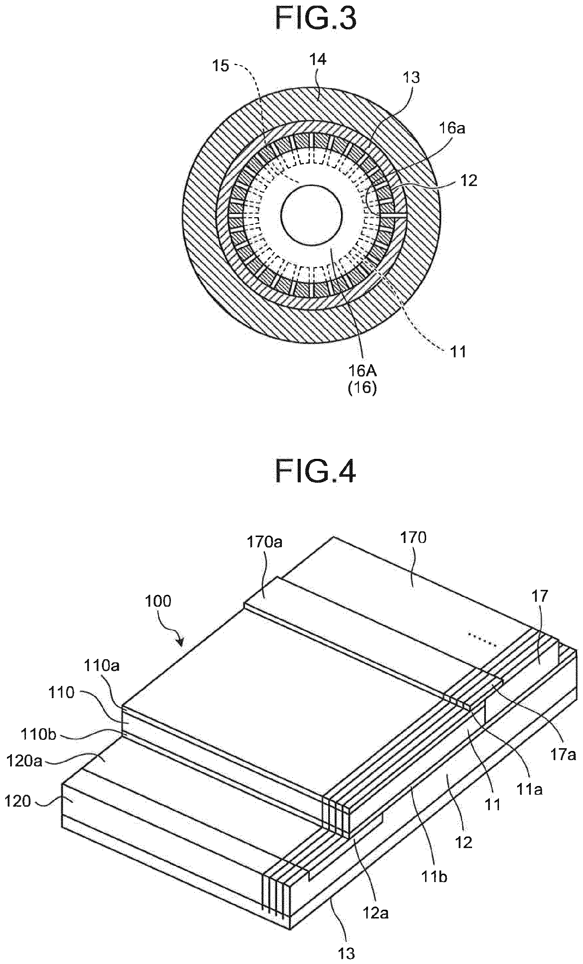

FIG. 3 is a diagram illustrating the configuration of the ultrasound transducer according to the embodiment, a diagram illustrating a cross section taken along line A-A in FIG. 2. The ultrasound transducer 10 includes a plurality of piezoelectric elements 11, a plurality of first acoustic matching layers 12, a second acoustic matching layer 13, an acoustic lens 14, a backing material 15, and a structural member 16. The plurality of piezoelectric elements 11 has a prismatic shape, being arranged in a circumferential direction, aligned in a longitudinal direction. The plurality of first acoustic matching layers 12 is provided on the inner periphery side of the piezoelectric elements 11. The second acoustic matching layer 13 has a substantially cylindrical shape, provided on the side (outer side surface side), opposite to the side coming in contact with the piezoelectric element 11, on the first acoustic matching layer 12. The acoustic lens 14 is provided on the side opposite to the side coming in contact with the first acoustic matching layer 12, on the second acoustic matching layer 13. The backing material 15 is provided on the opposite side of the side coming in contact with the first acoustic matching layer 12, on the piezoelectric element 11. The structural member 16 has a hollow disc shape and is provided for maintaining the shape of the ultrasound transducer 10. In the embodiments, the first acoustic matching layer 12 is provided for each of the piezoelectric elements 11, while the second acoustic matching layer 13 and the acoustic lens 14 integrally cover the plurality of piezoelectric elements 11 and the first acoustic matching layer 12. Furthermore, the configuration in the embodiment is such that the backing material 15 is filled into the portions between the piezoelectric elements 11. On the ultrasound transducer 10, one piezoelectric element 11 may be defined as a unit of output or the plurality of piezoelectric elements 11 may be defined as a unit of output.

The ultrasound transducer 10 is produced by rolling the sheet-shaped second acoustic matching layer 13 on which the plurality of piezoelectric elements 11 and the first acoustic matching layer 12 are arranged, to be deformed into a cylindrical shape, such that the piezoelectric element 11 is provided on an inner periphery side, then, arranging the structural member 16, thereafter, performing bonding by applying an adhesive to a gap between both ends of the first acoustic matching layer 12 and the second acoustic matching layer 13 in an arrangement direction of the piezoelectric elements 11, and filling the backing material 15. Details of the method for manufacturing the ultrasound transducer 10 will be described below.

The piezoelectric element 11 converts an electrical pulse signal into an ultrasound pulse (acoustic pulse), emits the ultrasound pulse to the subject, converts an ultrasound echo reflected from the subject into an electrical echo signal represented by a voltage change, and outputs the echo signal. An electrode 11a for signal input/output is provided on a backing material 15-side main surface on the piezoelectric element 11, and an electrode 11b for grounding is provided on a first acoustic matching layer 12-side main surface of the piezoelectric element 11 (refer to FIG. 4, etc.). Each of the electrodes 11a and 11b is formed using a metal material or a resin material, having conductivity. Moreover, the piezoelectric element 11 is electrically connected with a substrate 17 via an electrode 17a.

The piezoelectric element 11 is formed by PZT ceramic material, PMN-PT single crystal, PMN-PZT single crystal, PZN-PT single crystal, PIN-PZN-PT single crystal, or relaxer-based material. The PMN-PT single crystal is an abbreviation of a solid solution of lead magnesium niobate and lead titanate. The PMN-PZT single crystal is an abbreviation of a solid solution of lead magnesium niobate and lead zirconate titanate. The PZN-PT single crystal is an abbreviation of a solid solution of lead zinc-niobate and lead titanate. The PIN-PZN-PT single crystal is an abbreviation of a solid solution of lead indium niobate, lead zinc-niobate, and lead titanate. The relaxer-based material is a general term of a three-component piezoelectric material obtained by adding lead-based complex perovskite as a relaxer material is added to the lead zirconate titanate (PZT) for the purpose of increasing the piezoelectric constant and dielectric constant. The lead-based complex perovskite is represented by Pb(B1, B2)O₃, in which B1 is any of magnesium, zinc, indium, and scandium, while B2 is any of niobium, tantalum, and tungsten. These materials have excellent piezoelectric effects. These materials could reduce the value of the electrical impedance even in a miniaturized form, and thus, would be preferable from the viewpoint of impedance matching with a thin film electrode provided on the piezoelectric element 11.

In order to efficiently transmit the sound (ultrasound) between the piezoelectric element 11 and the observation target, the first acoustic matching layer 12 and the second acoustic matching layer 13 perform acoustic impedance matching between the piezoelectric element 11 and the observation target. The first acoustic matching layer 12 and the second acoustic matching layer 13 are formed of mutually different materials. The first acoustic matching layer 12 includes an electrode 12a electrically connected with the electrode 11b. In the first embodiment, there are two acoustic matching layers (first acoustic matching layer 12 and second acoustic matching layer 13). Alternatively, one layer may be provided or three or more layers may be provided in accordance with characteristics of the piezoelectric element 11 and the observation target.

The acoustic lens 14 is formed by silicone, polymethyl pentene, epoxy resin, polyetherimide, or the like. One of the surfaces of the acoustic lens 14 is formed into a protruding or recessed shape, leading to a function of narrowing the ultrasound, thereby emitting the ultrasound that passes through the acoustic matching layer to the outside, or incorporating an ultrasound echo from the outside. The acoustic lens 14 may be provided optionally. That is, the acoustic lens 14 may not be provided.

The backing material 15 attenuates unneeded ultrasound vibration generated by operation of the piezoelectric element 11. The backing material 15 is formed of a material having a high attenuation rate, for example, epoxy resin in which a filler such as alumina and zirconia is dispersed, or formed of a rubber in which the above-described filler is dispersed.

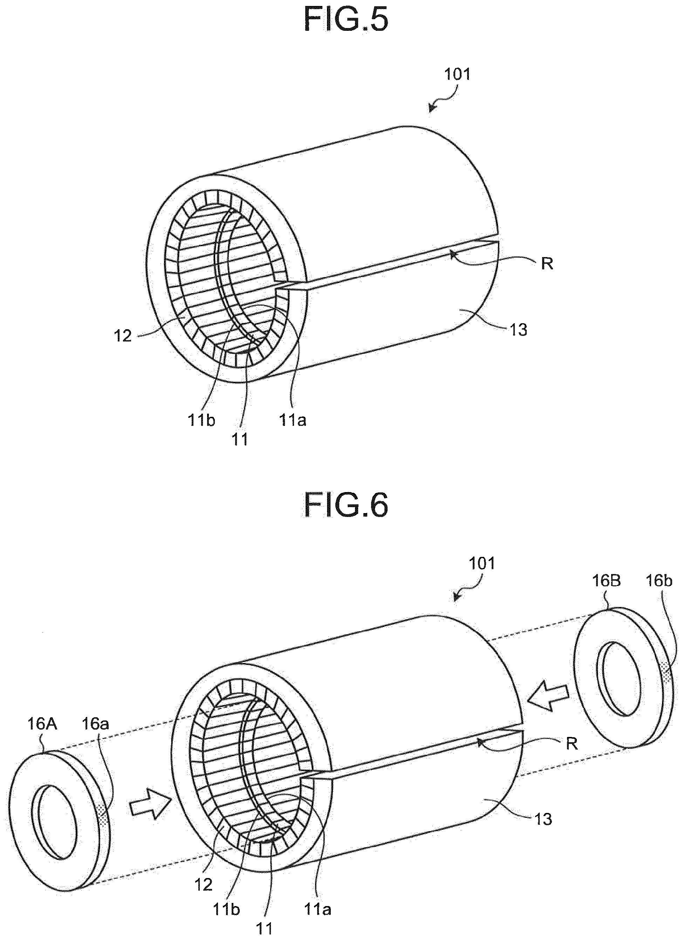

The structural member 16 has a hollow disc shape having an outer diameter corresponding to the diameter of a circle formed by the plurality of first acoustic matching layers 12 or corresponding to the diameter of a circle formed by the inner periphery of the plurality of substrates 17. Specifically, the structural member 16 includes a first structural member 16A and a second structural member 16B (refer to FIG. 6). The first structural member 16A is provided on one end side in a direction (longitudinal direction) orthogonal to a plane formed by the circumferential direction of the second acoustic matching layer 13. The second structural member 16B is provided on the other end side of the second acoustic matching layer 13 in the longitudinal direction. The first structural member 16A has a hollow shape having an outer diameter corresponding to the diameter of a circle formed by the plurality of first acoustic matching layers 12, one surface of which being covered with a conductive material such as copper foil. The second structural member 16B has a hollow disc shape having an outer diameter corresponding to the diameter of a circle formed by the inner periphery of the plurality of substrates 17.

Moreover, water repellent portions (16a and 16b, refer to FIG. 6) having fluorine coating are formed at a portion of the outer side surface of the structural member 16, that is, on an outer side surface facing a bonding portion (gap) at the ends of the first acoustic matching layer 12 and the second acoustic matching layer 13.

The piezoelectric element 11 vibrates with the input of the pulse signal, whereby the above-configured ultrasound transducer 10 emits ultrasound to the observation target via the first acoustic matching layer 12, the second acoustic matching layer 13, and the acoustic lens 14. At this time, the piezoelectric element 11 is configured such that the backing material 15 attenuates vibration of the piezoelectric element 11, thereby suppressing the transmission of vibration of the piezoelectric element 11, on the opposite side of the arrangement side of the first acoustic matching layer 12, the second acoustic matching layer 13, and the acoustic lens 14. Moreover, the ultrasound reflected from the observation target is transmitted to the piezoelectric element 11 via the first acoustic matching layer 12, the second acoustic matching layer 13, and the acoustic lens 14. The transmitted ultrasound causes the piezoelectric element 11 to vibrate, then, the piezoelectric element 11 converts the vibration into an electrical echo signal, and outputs, as an echo signal, the electrical echo signal to the ultrasound observation apparatus 3 via wiring (not illustrated).

Returning to FIG. 1, the operating unit 22 is coupled to the proximal end side of the insertion unit 21 and receives various types of operation from a doctor, or the like. As illustrated in FIG. 1, the operating unit 22 includes a bending knob 221 for performing bending operation on the bending portion 212, and a plurality of operating members 222 for performing various types of operation. Moreover, the operating unit 22 has a treatment tool insertion port 223 that communicates with the treatment tool insertion passage and that is used for inserting treatment tools into the treatment tool insertion passage.

Extending from the operating unit 22, the universal cable 23 includes a plurality of signal cables for transmitting various signals and an optical fiber for transmitting illumination light supplied from the light source device 6.

The connector 24 is provided at the distal end of the universal cable 23. The connector 24 includes first to third connector units 241 to 243 each of which being connected with an ultrasound cable 31, a video cable 41, and an optical fiber cable 61, respectively.

The ultrasound observation apparatus 3 is electrically connected with the ultrasound endoscope 2 via the ultrasound cable 31 (refer to FIG. 1), outputs a pulse signal to the ultrasound endoscope 2 via the ultrasound cable 31, while inputting echo signals from the ultrasound endoscope 2. Then, the ultrasound observation apparatus 3 performs predetermined processing on the echo signal and generates an ultrasound image.

The endoscopic examination apparatus 4 is electrically connected with the ultrasound endoscope 2 via the video cable 41 (refer to FIG. 1), and inputs an image signal from the ultrasound endoscope 2 via the video cable 41. Then, the endoscopic examination apparatus 4 performs predetermined processing on the image signal and generates an endoscopic image.

The display device 5 is formed by liquid crystal, organic electroluminescence (EL), or the like, and displays an ultrasound image generated by the ultrasound observation apparatus 3 and an endoscopic image generated by the endoscopic examination apparatus 4, or the like.

The light source device 6 is connected with the ultrasound endoscope 2 via the optical fiber cable 61 (refer to FIG. 1) and supplies illumination light for illuminating portions inside the subject, to the ultrasound endoscope 2 via the optical fiber cable 61.

Subsequently, a method for manufacturing the above-described ultrasound transducer 10 will be described with reference to FIGS. 4 to 15. First, processing of producing a molding member (molding member 100 described below) for forming the plurality of piezoelectric elements 11, the first acoustic matching layer 12, or the like, will be described. FIG. 4 is a diagram illustrating a method for manufacturing the ultrasound transducer according to the embodiment of the present invention, a diagram illustrating formation of the plurality of piezoelectric elements 11, the first acoustic matching layer 12, or the like.

First, a first thin film 110a formed of a material for constituting an electrode 11a, and a second thin film 110b formed of a material for constituting an electrode 11b are stacked onto opposing main surfaces of a cuboid-shaped piezoelectric element base material 110 formed of a material for constituting the piezoelectric element 11. Thereafter, the piezoelectric element base material 110 and a substrate base material 170 formed of a material for constituting the substrate 17, are arranged side by side, then, the two base materials are connected by an electrode material 170a formed using a material for constituting the electrode 17a. In a state where the piezoelectric element base material 110 and the substrate base material 170 are arranged side by side, a first acoustic matching layer base material 120 formed of a material for constituting the first acoustic matching layer 12 (including a material 120a for constituting the electrode 12a) is stacked on a side of the second thin film 110b, on the side opposite to the piezoelectric element base material 110 side, while the sheet-shaped second acoustic matching layer 13 is stacked on a side of the first acoustic matching layer base material 120, on the side opposite to the second thin film 110b side. The molding member 100 illustrated in FIG. 4 is produced by the above-described stacking processing.

Thereafter, using a blade, such as a dicing saw, for the molding member 100, the piezoelectric element base material 110, the first thin film 110a, the second thin film 110b, and the first acoustic matching layer base material 120 are cut at a predetermined pitch. With this process, it is possible to form the plurality of piezoelectric elements 11 (including the electrodes 11a and 11b) and the first acoustic matching layer 12 (including the electrode 12a), on the sheet-shaped second acoustic matching layer 13.

FIG. 5 is a diagram illustrating a method for manufacturing the ultrasound transducer according to the embodiment of the present invention. When the second acoustic matching layer 13 on which the plurality of piezoelectric elements 11 and the first acoustic matching layer 12 are formed is deformed into a cylindrical shape along the arrangement direction of the piezoelectric elements 11, it is possible to obtain a cylindrical shaped structure 101 configured such that the piezoelectric element 11 is arranged on the inner periphery side and the second acoustic matching layer 13 is arranged on the outer periphery side, as illustrated in FIG. 5.

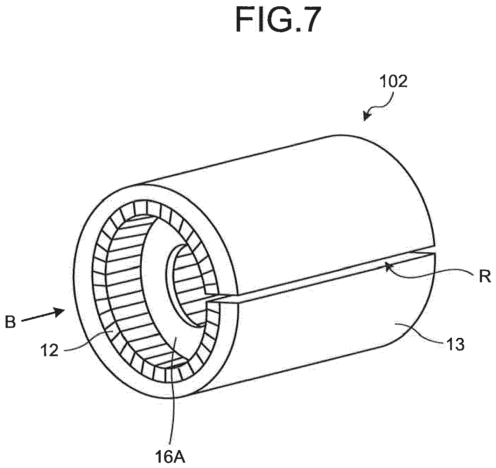

FIGS. 6 and 7 are diagrams each illustrating a method for manufacturing the ultrasound transducer according to the embodiment of the present invention, a diagram illustrating attachment of the structural member 16. By arranging the structural member 16 inside the cylindrically-deformed structure, it is possible to stabilize the shape of the ultrasound transducer 10. As illustrated in FIG. 6, the first structural member 16A is inserted at an end on the side where the piezoelectric element 11 is exposed, while the second structural member 16B is inserted at an end on the side where the substrate 17 is exposed. At this time, insertion of the first structural member 16A and the second structural member 16B is performed such that the water repellent portions 16a and 16b of the first structural member 16A and the second structural member 16B face the bonding portion at the ends of the first acoustic matching layer 12 and the second acoustic matching layer 13. With this process, it is possible to obtain a structure 102 (refer to FIG. 7) in which the first structural member 16A and the second structural member 16B are arranged on both ends of the structure 101. Alternatively, the second acoustic matching layer 13 may be wound around the structural member 16 to integrate, thereby producing the structure 102. The first structural member 16A is arranged such that a conductive surface thereof is exposed.

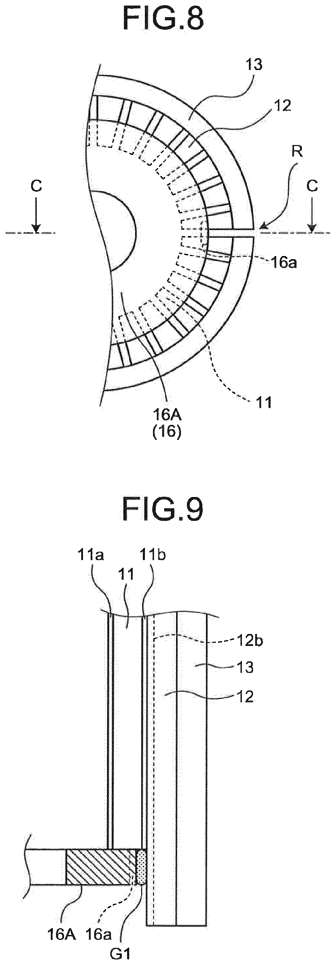

FIG. 8 is a diagram illustrating a method for manufacturing the ultrasound transducer according to the embodiment of the present invention, a diagram illustrating a configuration of a portion of the structure 102 viewed from the arrow B direction in FIG. 7. In this case, the portion includes the bonding portion at the ends of the first acoustic matching layer 12 and the second acoustic matching layer 13. FIG. 9 is a diagram illustrating a method for manufacturing the ultrasound transducer according to the embodiment of the present invention, a cross-sectional view taken along line C-C in FIG. 8.

As illustrated in FIG. 8, the water repellent portion 16a faces a gap R between the both ends in the circumferential direction of the first acoustic matching layer 12 and the second acoustic matching layer 13, and is in contact with a part of the first acoustic matching layer 12. Moreover, as illustrated in FIG. 9, a masking material 12b is provided at a part of a surface forming the gap R of the first acoustic matching layer 12, that is, at an inner periphery side edge of the first acoustic matching layer 12. The masking material 12b is provided at a stacking-side edge of the piezoelectric element 11, for example, before forming the structure 101, for example, when the molding member 100 is produced. The masking material 12b is formed of a silicone rubber for modeling, for example.

Moreover, the first structural member 16A is fixed onto the first acoustic matching layer 12 by introducing an adhesive G1 from the outside of the structure 102 and solidifying the adhesive G1. The adhesive G1 is not flown into the water repellent portion 16a having high water repellency, and thus, the adhesive G1 is not arranged at the water repellent portion 16a when the first structural member 16A is bonded to the first acoustic matching layer 12. In other words, the first structural member 16A is bonded to the first acoustic matching layer 12 on a side surface other than the portion where the water repellent portion 16a is formed, among the side surfaces of the disc. Herein, the side surface represents a surface different from a surface having the largest area (main surface).

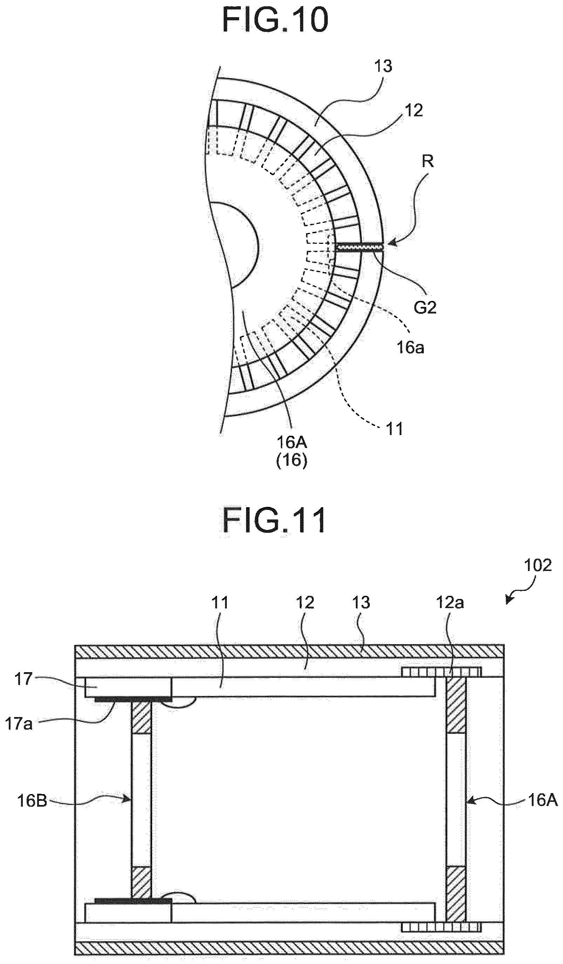

FIG. 10 is a diagram illustrating a method for manufacturing the ultrasound transducer according to the embodiment of the present invention, a diagram illustrating a configuration in which an adhesive G2 is provided at the gap R of the first acoustic matching layer 12 and the second acoustic matching layer 13. FIG. 10 is a diagram corresponding to the arrow B direction in FIG. 7. As illustrated in FIG. 10, the ends of the first acoustic matching layer 12 and the second acoustic matching layer 13 in the circumferential direction are fixed with each other, by introducing the liquid adhesive G2 into the gap R of the first acoustic matching layer 12 and the second acoustic matching layer 13 to solidify the adhesive G2. At this time, since the masking material 12b is provided at the first acoustic matching layer 12, the liquid adhesive G2 is not going to flow into the piezoelectric element 11.

In a case where the water repellent portion 16a is not provided on the structural member 16A, the adhesive G2 may flow from a gap between the structural member 16A and the first acoustic matching layer 12, into the adjacent piezoelectric elements 11, due to capillary phenomenon. In contrast, since the water repellent portion 16a is provided on the structural member 16A in the embodiment, water repellent action suppresses the occurrence of capillary phenomenon even if the above-described gap exists, which makes it possible to prevent the adhesive G2 from flowing between the piezoelectric elements 11.

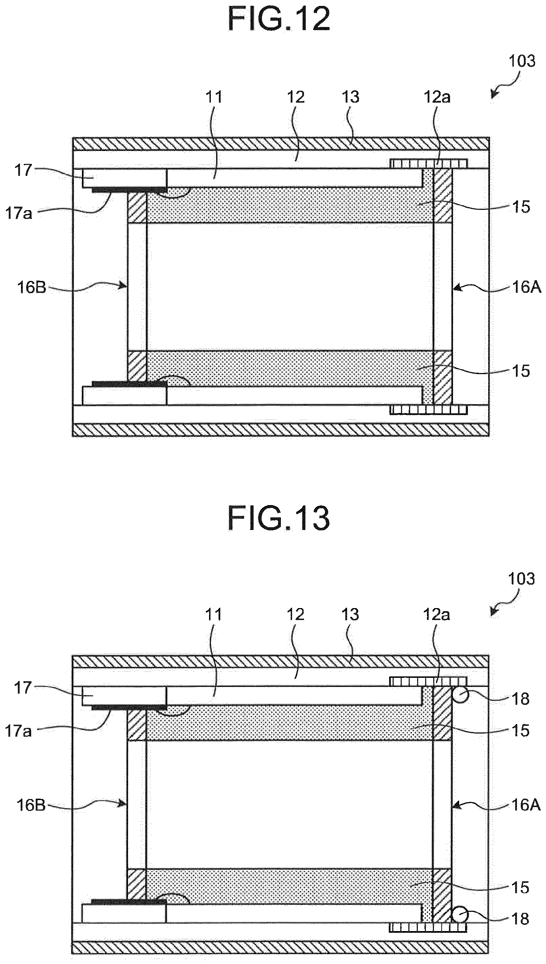

FIG. 11 is a diagram illustrating a method for manufacturing the ultrasound transducer according to the embodiment of the present invention, a diagram illustrating the structure 102 before the backing material 15 is filled. FIG. 12 is a diagram illustrating a method for manufacturing the ultrasound transducer according to the embodiment of the present invention, a diagram illustrating a structure 103 after the backing material 15 is filled. A liquid backing material constituting the backing material 15 is introduced into a space formed by the structural member 16, onto the structure 102 illustrated in FIG. 11, and the material for the backing material is solidified, thereby forming the backing material 15 on the side opposite to the first acoustic matching layer 12 side of the piezoelectric element 11, and on portions between the piezoelectric elements 11.

At this time, since the adhesive G2 has not been flown into the portions between the piezoelectric elements 11 as described above, it is possible to introduce and fill the backing material 15 between the piezoelectric elements 11.

FIG. 13 is a diagram illustrating a method for manufacturing the ultrasound transducer according to the embodiment of the present invention. After formation of the backing material 15, a conductive member 18 electrically connecting the surface of the first structural member 16A and the electrode 12a is provided. This enables the electrode 12a to be electrically connected to an external circuit (for example, ground) via the first structural member 16A.

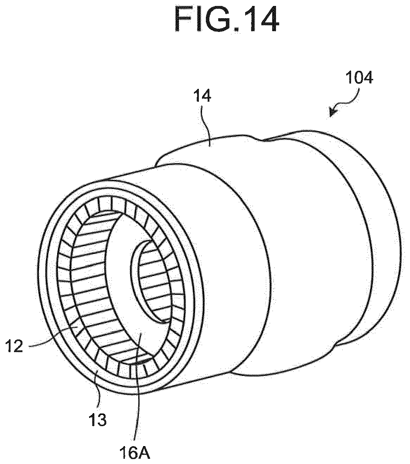

FIG. 14 is a diagram illustrating a method for manufacturing the ultrasound transducer according to the embodiment of the present invention, a diagram illustrating attachment of the acoustic lens 14. After producing the structure 103 by providing the structure 102 with the backing material 15 and the conductive member 18, the acoustic lens 14 is provided on an external side surface of the second acoustic matching layer 13, as illustrated in FIG. 14, thereby producing a structure 104.

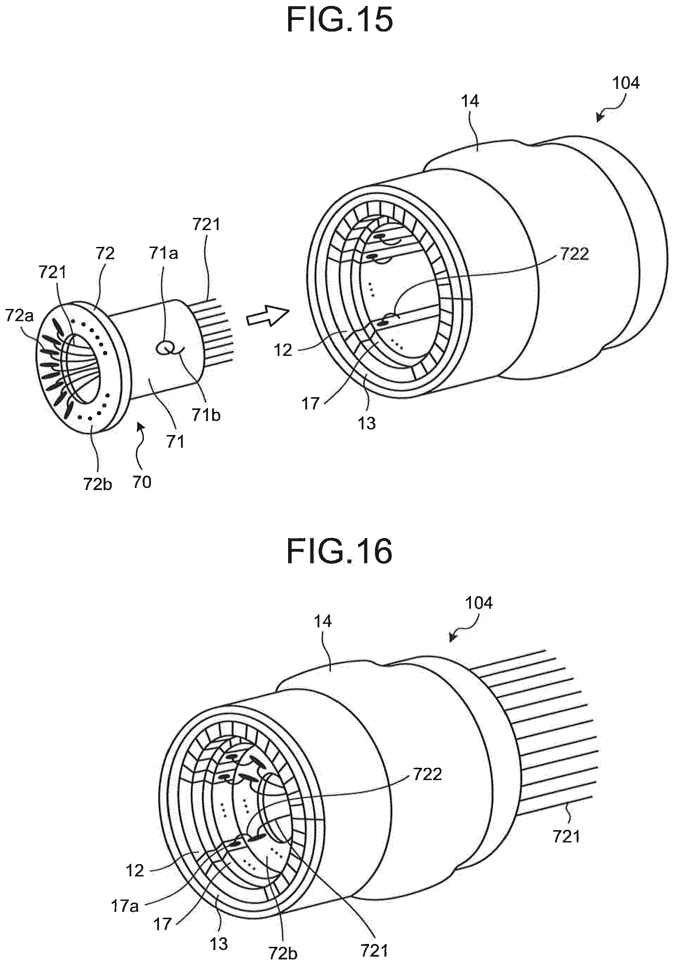

FIGS. 15 and 16 are diagrams each illustrating a method for manufacturing the ultrasound transducer according to the embodiment of the present invention, a diagram illustrating attachment of a wiring member 70 onto the structure 104. After production of the structure 104, the substantially cylindrical shaped wiring member 70 is inserted from one opening portion side of the structure 104, that is the side on which the substrate 17 is provided, as illustrated in FIG. 15. The wiring member 70 includes a cylindrical-shaped cylinder portion 71 and a flange portion 72 provided at one end of the cylinder portion 71, having an external diameter greater than the diameter of the cylinder portion 71. The surface of the flange portion 72 includes a printed circuit board 72b on which several tens to several hundreds of electrode pads 72a are formed.

A bundle of cables 721 is inserted through an inner portion of the cylinder portion 71, and one end of each of the cables 721 is soldered with each of the electrode pads 72a (cable 721 is soldered onto the inner side portion (center direction of the ring) of the electrode pad 72a to be connected). For noise reduction, a coaxial cable is normally used as the cable 721.

The cylinder portion 71 is formed by insulating material (e.g., engineering plastic) and has a plating of conductor (metal thin film) on the surface. Examples of insulating materials include polysulfone, polyetherimide, polyphenylene oxide, and epoxy resins. On the surface of the cylinder portion 71, a hole 71a is formed to penetrate from the inner periphery to the outer periphery. A ground line 71b extending from the bundle of cables inserted through the wiring member 70 further extends from a hole 71a, to be joined with the metal thin film plated on the outer side surface of the cylinder portion 71.

When the wiring member 70 connecting with the cable 721 is inserted into the structure 104, the flange portion 72 is abutted against the second structural member 16B of the structure 104, then, the wiring member 70 is positioned inside the structure 104.

After insertion and positioning of the wiring member 70 are performed, an outer periphery side portion (electrode pad portion in outer peripheral direction of the ring) of the electrode pad 72a is connected with the electrode 17a of the substrate 17 using a wire 722, as illustrated in FIG. 16. With this process, it is possible to obtain the ultrasound transducer 10 that is electrically connected to the cable 721. Thereafter, the balloon locking unit 211b is attached so as to produce the rigid member 211 together with the proximal end portion 211a.

According to the above-described embodiment, the water repellent portions 16a and 16b are formed corresponding to the gap R formed by the end portions in the circumferential direction on the cylindrical-shaped acoustic matching layer, on the first structural member 16A and the second structural member 16B of the ultrasound transducer 10, thereby suppressing the capillary phenomenon that occurs at a gap between the structural member 16 and the first acoustic matching layer 12, formed by the adhesive G2. This configuration prevents the adhesive G2 from flowing into the portion between the piezoelectric elements 11, and enables the backing material 15 to be filled into the portion between the piezoelectric elements 11, making it possible to suppress deterioration in acoustic performance in the radial ultrasound transducer.

Moreover, by providing the water repellent portions 16a and 16b in a smallest region possible to suppress capillary phenomenon, on the bonding portion at the end of the acoustic matching layer in the above-described embodiment, it is possible to ensure a large region to which the adhesive G1 is applicable, on portions other than the water repellent portions 16a and 16b, on the first structural member 16A and the second structural member 16B. Accordingly, it is possible to fix the structural member 16 onto the first acoustic matching layer 12, or onto the substrate 17 further tightly. Therefore, it is preferable to have a formation region of the water repellent portions on the outer side surface of the structural member 16, to be smaller than the region other than the water repellent portions. It would be more preferable that the water repellent portions 16a and 16b are at least arranged in accordance with the portion between the ends of the acoustic matching layer and that the portions are formed in a smallest possible region.

Moreover, since the above-described embodiment forms the water repellent portions 16a and 16b using a thin film with fluorine coating, it is possible to reduce the radial length of the structural member 16 compared with the water repellent treatment such as water repellent sealing.

### (Modification of Embodiments)

Although the water repellent portions 16a and 16b are formed using fluorine coating in the above-described embodiment, alternative structure may be employed as long as super-water repellent processing can be performed on the structural member 16. For example, hydrophobic silica coating or a double roughness structure may be employed. Specifically, the double roughness structure means a surface roughness structure having a plurality of micrometer-size projections (concave-convex shape) including nanometer-size projections. Water repellent coating such as fluorine coating or hydrophobic silica coating is more easily manufactured with lower cost compared with the double roughness structure, while the double roughness structure has an advantage of higher water repellency compared with the water repellent coating. The "super-water repellent" is a phenomenon of a water droplet being in contact with a water droplet holding surface with a contact angle greater than 150°.

Moreover, instead of the masking material 12b, super-water repellent processing, such as fluorine coating, hydrophobic silica coating, and double roughness structure, may be employed.

Moreover, two structural members (first structural member 16A and second structural member 16B) are provided in the above-described embodiments. Alternatively, only one of the first structural member 16A and the second structural member 16B may be provided.

Embodiments of the present invention have been described hereinabove, however, the present invention is not intended to be limited to the above-described embodiments and the modification. In this manner, the present invention is not intended to be limited to the above-described embodiments and modification but may include various forms of embodiments without deviating from the technical spirit and scope of the general inventive concept as defined in the appended claims of this invention. Furthermore, the elements described in each of the embodiments and modifications may be appropriately combined with each other.

As the ultrasound probe, an ultrasound miniature probe that has a small diameter and has no optical system may be employed. In typical cases, the ultrasound miniature probe is inserted into biliary tract, bile duct, pancreatic duct, trachea, bronchus, urethra, and ureter to observe the nearby organs (pancreas, lung, prostate gland, bladder, and lymph nodes, or the like).

Alternatively, an external ultrasound probe for emitting ultrasound from a body surface of the subject may be employed as the ultrasound probe. The external ultrasound probe is typically used to observe abdominal organs (liver, gall bladder, and bladder), breast (mammary gland, in particular), and the thyroid.

### Industrial Applicability

As described hereinabove, the ultrasound probe according to the present invention is useful in suppressing deterioration in acoustic performance of the radial ultrasound transducer.

### Reference Signs List

- 1: ENDOSCOPE SYSTEM
- 2: ULTRASOUND ENDOSCOPE
- 3: ULTRASOUND OBSERVATION APPARATUS
- 4: ENDOSCOPIC EXAMINATION APPARATUS
- 5: DISPLAY DEVICE
- 6: LIGHT SOURCE DEVICE
- 10: ULTRASOUND TRANSDUCER
- 11: PIEZOELECTRIC ELEMENT
- 12: FIRST ACOUSTIC MATCHING LAYER
- 13: SECOND ACOUSTIC MATCHING LAYER
- 14: ACOUSTIC LENS
- 15: BACKING MATERIAL
- 16: STRUCTURAL MEMBER
- 16A: FIRST STRUCTURAL MEMBER
- 16B: SECOND STRUCTURAL MEMBER
- 16a, 16b: WATER REPELLENT PORTION
- 17: SUBSTRATE
- 21: INSERTION UNIT
- 22: OPERATING UNIT
- 23: UNIVERSAL CABLE
- 24: CONNECTOR
- 31: ULTRASOUND CABLE
- 41: VIDEO CABLE
- 61: OPTICAL FIBER CABLE

## Claims

1. An ultrasound probe comprising:
an acoustic matching layer having a sheet-shaped member whose both ends are bonded together by an adhesive to define a cylindrical shape and configured to adjust acoustic impedance of ultrasound;
a plurality of piezoelectric elements arranged along a circumferential direction on an inner periphery of the acoustic matching layer and configured to emit ultrasound in response to an input of an electrical signal and configured to convert ultrasound that has entered from outside, into an echo signal;
a structural member having a hollow disc shape and provided on at least one end side of the acoustic matching layer in a longitudinal direction orthogonal to the circumferential direction, at least a part of an outer side surface of the structural member being fixed inside the acoustic matching layer,
wherein the structural member has a water repellent portion on a part of the outer side surface such that the water repellent portion faces, in a radial direction, a gap between the both ends of the acoustic matching layer.

2. The ultrasound probe according to claim 1, further comprising a second structural member having a hollow disc shape and provided on the other end side in the longitudinal direction of the acoustic matching layer, an outer side surface of the second structural member being fixed inside the acoustic matching layer,
wherein the second structural member has a water repellent portion on the outer side surface so as to face the gap in the radial direction.

3. The ultrasound probe according to claim 1 or 2,
wherein the water repellent portion is formed by one of a surface having water repellent coating or a surface having a surface roughness structure.

4. The ultrasound probe according to claim 3,
wherein the water repellent coating is one of fluorine coating and hydrophobic silica particle coating.

5. The ultrasound probe according to claim 3,
wherein the surface roughness structure is a double roughness structure.

6. The ultrasound probe according to claim 1,
wherein the water repellent portion is exposed to outside by the gap.

7. The ultrasound probe according to claim 1,
wherein the structural member is configured such that the outer side surface other than the water repellent portion is bonded to the acoustic matching layer via an adhesive.
